# EUROPEAN PATENT SPECIFICATION

(11) **EP 3 166 406 B1**
(45) Date of publication and mention of the grant of the patent: **05.08.2020**
(21) Application number: 15800453.1
(22) Date of filing: 01.06.2015
(51) Int. Cl.: A01N 43/04, A61K 31/715, A61K 38/20, A61K 45/06, A61P 35/00, A61P 37/04, A61P 37/08, A61P 37/02

(54) **CYTOKINE-CHITOSAN BIOCONJUGATES AND METHODS OF USING THE SAME**
CYTOKIN-CHITOSAN-BIOKONJUGATE UND VERFAHREN ZUR VERWENDUNG DERSELBEN
BIOCONJUGUÉS DE CHITOSANE-CYTOKINE ET PROCÉDÉS POUR LES UTILISER

(30) Priority: 31.05.2014 US 201462006114 P
(43) Date of publication of application: 17.05.2017
(73) Proprietor: The Board of Trustees of the University of Arkansas, Little Rock, AR 72207 (US)
(72) Inventor: ZAHAROFF, David, A., Fayetteville, AR 72703 (US); THALLAPURANAM, Suresh, Kumar, Fayetteville, AR 72704 (US); JAYANTHI, Srinivas, Fayetteville, AR 72701 (US); KOPPOLU, Bhanu, Prasanth, Cary, NC 27513 (US); SMITH, Sean, C., Cary, NC 27513 (US)
(74) Representative: Mewburn Ellis LLP
(86) International application number: PCT/US2015/033541
(87) International publication number: WO 2015/184445

(56) References cited:
- WO-A1-2009/123713
- WO-A2-2008/039390
- KR-A- 20070 050 586
- US-A1- 2007 031 468
- US-A1- 2007 292 387
- US-A1- 2010 291 043
- US-A1- 2012 107 268
- US-A1- 2013 129 674
- DAVID A. ZAHAROFF ET AL: "Intratumoral Immunotherapy of Established Solid Tumors With Chitosan/IL-12 :", JOURNAL OF IMMUNOTHERAPY, vol. 33, no. 7, 1 September 2010 (2010-09-01), pages 697-705, XP055422609, ISSN: 1524-9557, DOI: 10.1097/CJI.0b013e3181eb826d
- SASAMON SUPAPRUTSAKUL ET AL: "Transfection efficiency of depolymerized chitosan and epidermal growth factor conjugated to chitosan-DNA polyplexes", JOURNAL OF MATERIALS SCIENCE: MATERIALS IN MEDICINE, KLUWER ACADEMIC PUBLISHERS, BO, vol. 21, no. 5, 26 January 2010 (2010-01-26), pages 1553-1561, XP019822031, ISSN: 1573-4838
- HEFFERNAN M J ET AL: "In vivo efficacy of a chitosan/IL-12 adjuvant system for protein-based vaccines", BIOMATERIALS, ELSEVIER SCIENCE PUBLISHERS BV., BARKING, GB, vol. 32, no. 3, 1 January 2011 (2011-01-01), pages 926-932, XP027506453, ISSN: 0142-9612, DOI: 10.1016/J.BIOMATERIALS.2010.09.058 [retrieved on 2010-10-20]
- VINSOVA ET AL.: 'Recent Advances in Drugs and Prodrugs Design of Chitosan' CURRENT PHARMACEUTICAL DESIGN vol. 14, no. 13, 2008, ISSN 1381-6128 pages 1311 - 1326, XP055238620

## Description

### CROSS-REFERENCE TO RELATED APPLICATIONS

This patent application claims the benefit of priority of United States Provisional Patent Application No. 62/006,114, filed May 31, 2014.

### STATEMENT REGARDING FEDERALLY SPONSORED RESEARCH

This invention was made with United States government support awarded by the National Institutes of Health grant number P30 GM103450 and R01CA172631. The United States has certain rights in this invention.

### INTRODUCTION

Cytokines are powerful modulators of immune function. For decades, scientists have hypothesized that exogenous cytokines can be administered to overcome immune dysfunction and treat a wide variety of diseases, including cancer. However, despite hundreds, if not thousands, of preclinical and clinical studies, only 2 of 40+ identified cytokines have been approved as single agent immunotherapies for a limited number of malignancies. Unintentional signaling and dose-limiting side effects due to systemic delivery of pleiotropic cytokines have prevented cytokine-based immunotherapies from fulfilling their clinical potential.

Zaharoff et al. 2010 (Journal of Immunotherapy, 33(7): 697-705) discloses the intratumoral administration of IL-12 cofomulated with the biodegradable polysaccharide chitosan and reports that chitosan/IL-12 immunotherapy increases local retention of IL-12 in the tumor microenvironment, eradicated established, aggressive murine tumors, and generated systemic tumor-specific protective immunity.

WO 2008/039390 A2 discloses methods and compositions related to chitosan antigen depots, and chitosan cytokine depots, and the use of depot compositions in treating and preventing diseases.

### SUMMARY

Compositions comprising chitosan covalently linked to IL-12 as set out in the claims are provided herein. The IL-12 in the composition is biologically active. Additionally disclosed herein are compositions comprising chitosan covalently linked to cytokines, including all of the interleukins and further include, but are not limited to, IL-2, IL-12, GM-CSF, 1L-1, TNF-α, JEFN-γ, IFN-α, IL-10, TGF-β, IL-15, IL-23, IL-27, IL-35 and IL-7. The cytokines or growth factors may be attached to the chitosan in a variety of ways and may contain mutations to allow covalent attachment to the chitosan. The chitosan may have a molecular weight between 10kDa and 500kDa, between 100kDa and 400kDa or between 200kDa and 300kDa. The compositions may be formulated into pharmaceutical compositions. The pharmaceutical compositions may be formulated for local administration.

Also provided are compositions for use in a method of treating a disorder in a subject, as set out in the claims. The compositions are administered to the subject in an amount effective to treat the disorder. The compositions may be administered locally. In one aspect, the disorder is cancer and the composition is administered intratumorally. In other embodiments, the disorder is an autoimmune disease, allergy or infection and the composition is formulated to target these disorders.

Also provided are compositions for use in a method of stimulating an immune response in a subject, as set out in the claims. The method comprises administering the compositions provided herein with an antigen to the subject in an amount effective to stimulate an immune response directed to the antigen. The antigen may be a protein, polypeptide or vaccine. The cytokine or growth factor chitosan bioconjugate may act as an adjuvant to further stimulate an immune response to the antigen.

### BRIEF DESCRIPTION OF THE DRAWINGS

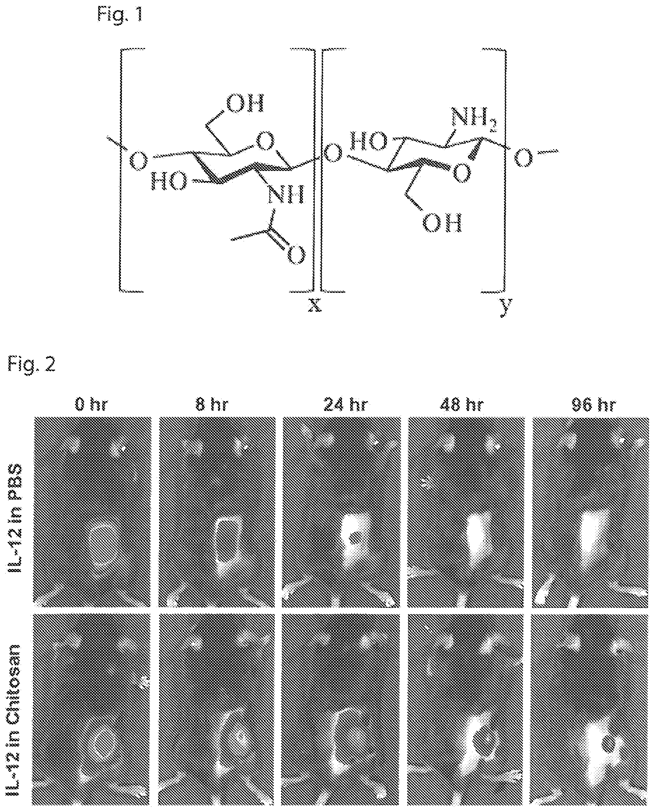

Figure 1 is a diagram showing the chemical structure of chitosan. Chitosan is an unbranched copolymer of N-acetylglucosamine (x) and glucosamine (y) units linked by β(1-4) glycosidic bonds where the ratio ofy to x is greater than 1:5
Figure 2 is a set of photographs showing that attachment to chitosan enhances cytokine retention in a subject. Mice bearing 7-day-old s.c MC32a (colon adenocarcinoma) tumors were shaved and given a single i.t. injection of Alexa Fluor 660(AP660)-labeled IL-12 (5µg) in either phosphate buffered saline (PBS) or 1.5% (w/v) chitosan solution. IL-12 became undetectable between 24 and 48 hrs when administered with PBS. When mixed with chitosan solution, IL-1.2 persisted in the tumor for at least 5 to 6 days. Method adapted from ref. [6].
Figure .3 is a graph showing that chitosan/IL-12 immunotherapy improves survival following breast tumor resection. Mice bearing orthotopic 4T1 mammary carcinomas were given i.t. treatments on days 6 and 12 after tumor implantation with (■) saline, (▲)chitosan, (●) IL-1 2 alone(1µg) or (○) chitosan/IL-12(1µg) admixture. Primary tumors were resected on day 15.

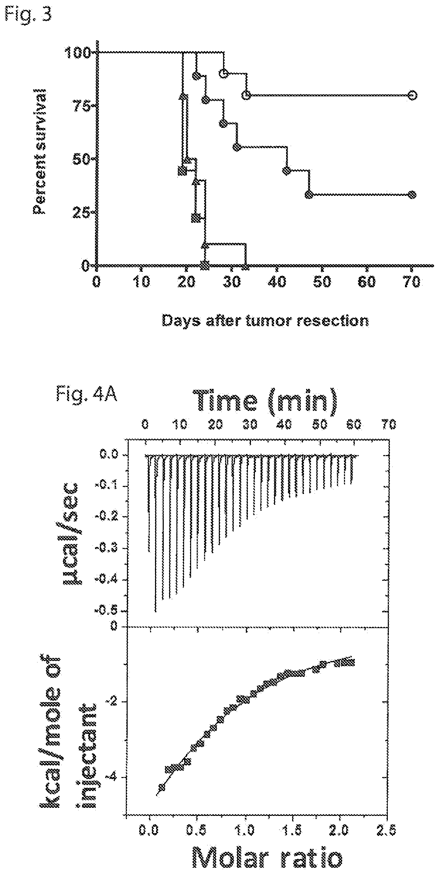

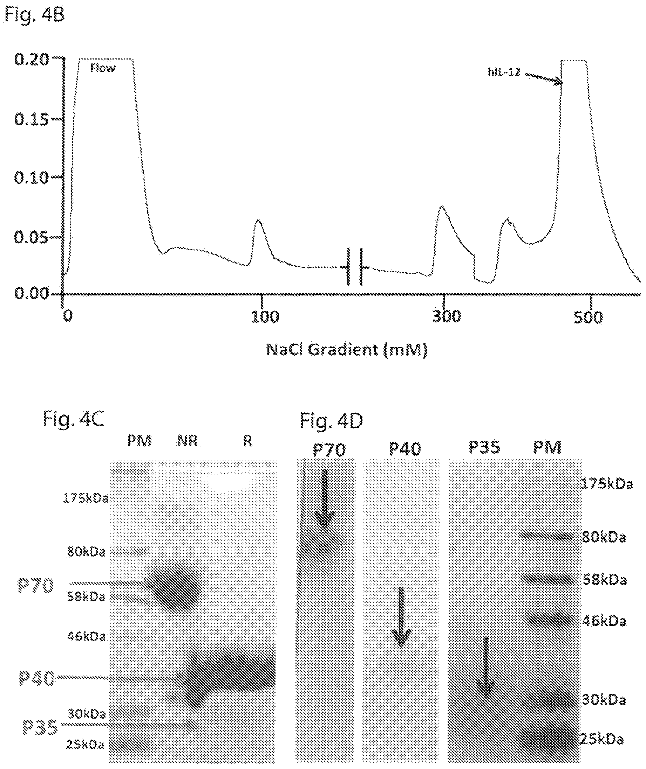

Figure 4 is a set of figures showing purification of recombinant human IL-12. Figure 4A is a set of isothermogram graphs depicting the heparin binding affinity of human IL-12. Figure 4B is a graph showing the protein purification profile on heparin-sepharose. Figure 4C and Figure 4D are photographs of SDS-PAGE gels of the 500 mM NaCl fraction stained with Coomassie Blue (Figure 4C) and detected with antibodies specific for the p70, p40 and p35 subunits of IL-12, respectively (Figure 4D). Lanes, **NR** and **R** represent the electrophoresis profile of IL-12 under non-reducing and reducing conditions, respectively. The minor additional bands represent different glycosylated products of IL-12.

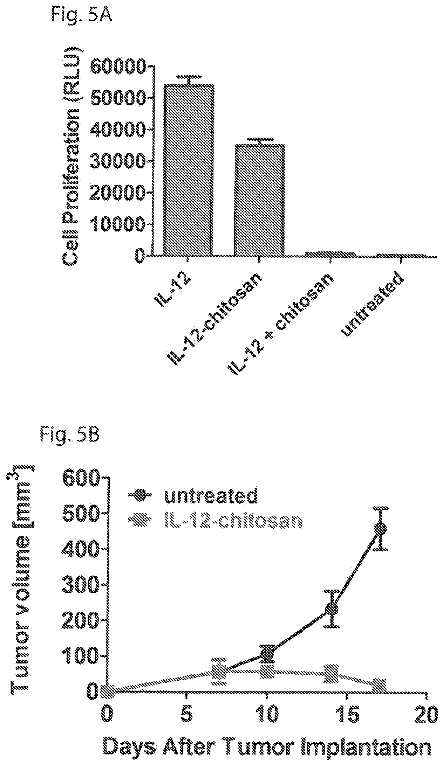

Figure 5 is a set of graphs showing the bioactivity of IL-12,chitosan bioconjugates. Figure 5A is a graph showing proliferation of IL-12-sensitive 2D6 cells treated with recombinant IL-12, IL-12-chitosan bioconjugates or unreacted, dialyzed IL-12+chitosan or nothing (untreated). The proliferation was measured via a luminescence-based cell viability assay (CellTiter-Glo; Promega). Figure 5B is a graph showing the change in tumor volume over time in C57BL/6 mice (n=4) inoculated s.c. with 500,000 MB49 bladder carcinoma cells. The mice were treated i.t. on days 7, 10 and 14 with IL-12-chitosan bioconjugates containing an estimated 1 µg IL-12. MB49-bearing control mice (n=5) were not treated.

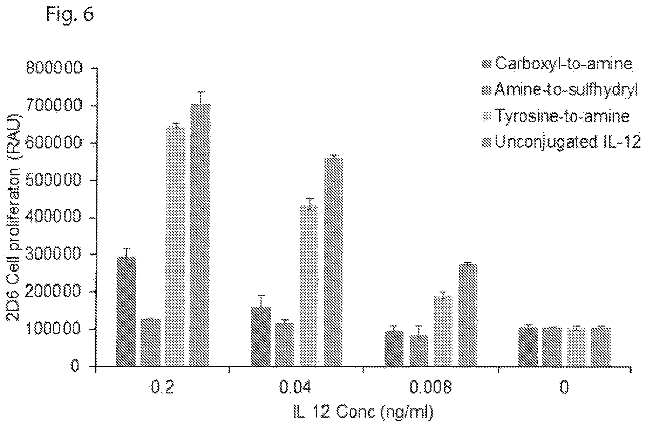

Figure 6 is a graph showing the bioactivity of IL-12-chitosan non-specific conjugates as quantified through IL-12 dependent 2D6 T cell proliferation assays. Unmodified IL-12 was non-specifically conjugated to chitosan following various bio-conjugation methods. Methods followed include, 1) carbodiimide mediated peptide bonding where amine groups on chitosan are covalently bonded to carboxylic acid groups on IL-12. 2) Amine-to- sulfhydryl crosslinker (Sulfo -SMCC ester) mediated conjugation of thiolated chitosan to IL-12, and 3) Tyrosinase mediated catalytic conjugation of tyrosinase modified o-quinones on IL-12 to nucleophilic amine group on chitosan. As seen, nonspecific conjugation of IL-12 to chitosan is effective to varying degree with amine-to- sulfhydryl crosslinker mediated conjugation yielding a completely inactive product. Peptide bonding and tyrosinase catalysis methods yielded chitosan-IL-12 conjugates that are bioactive with up to 60% and 17% loss of lL-12 bioactivity observed with peptide bonding and tyrosinase catalysis respectively.

### DETAILED DESCRIPTION

We hypothesized that a safer, more effective approach for the administration of cytokine therapeutics is through local, sustained delivery. Local delivery reduces cytokine-mediated toxicities and exploits the preferred paracrine mechanism of action of most cytokines.

Our recently published data demonstrate that intra-tumoral (i.t.) administration of IL-12 admixed with chitosan solutions (chitosan/IL-12) can retain IL-12 locally in the tumor and eradicate 80-100% of established colorectal, pancreatic and bladder tumors (Zaharoff et al. 2009; Zaharoff et al 2010; Yang et al 2013). Perhaps more important than primary tumor regression, was the finding that 47-100% of tumor-free mice rejected tumor rechallenge which implies the generation of tumor-specific immunity following chitosanlIL-12 immunotherapy. The use of intravesically administered chitosan/IL-12 admixtures for the treatment of superficial bladder cancer is under consideration.

Regarding breast cancer, recent preliminary data indicate that chitosan/IL-12 admixtures can control metastases and improve survival in an aggressive preclinical model. In these studies, mice bearing 4T1 tumors were given 3 i.t injections prior to primary tumor resection. Mice were followed for survival after resection. In a separate study, mice were euthanized five weeks after resection to document pulmonary metastases. In both cases, pre-resection treatment with chitosan/IL-12 improved surgical outcomes. Neoadjuvant immunotherapy with chitosan/IL-12 resulted in 67% durable cures. In contrast, none of the mice receiving tumor resection alone survived beyond 40 days after surgery.

Despite these promising results, systemic dissemination of IL-12 and the associated potential for toxicity remains a serious concern and a potential obstacle to clinical translation. The enhanced i.t retention of IL-12 is due to the fact that highly viscous chitosan solutions hinder the diffusive transport of IL-12. Nevertheless, the majority of administered IL-12 is likely to reach the systemic circulation. In fact, elevated serum IL-12 and IFN-γ levels following chitosan/IL-12 injections have confirmed the leakage of IL-12 from a local injection site.

To overcome this limitation, we propose a novel delivery technology in which IL-12 is covalently conjugated to chitosan prior to intratumoral (i.t) injection. Conjugation will increase the effective molecular weight of IL-12 and therefore reduce its diffusivity. In addition, polycationic chitosan is expected to interact electrostatically with negatively charged extracellular matrix proteins and cell membranes. Thus, IL-12-chitosan bioconjugates are effectively anchored to a local injection site. Similar methods may be useful with other cytokines or growth factors as well. Cytokine immunotherapy has been limited by significant toxicity or unintended side effects associated with systemic administration of the cytokines. Thus, conjugating the cytokine to chitosan to limit diffusion of the cytokine from the point of administration or injection may limit side effects or toxicity associated with cytokine administration.

In the Examples, 1L-12 was covalently linked to chitosan via a carbodiimide cross-linking between amine groups on the chitosan and carboxyl groups on the IL-12. While this form of non-specific linkage did reduce the activity of the IL-12, substantial IL-12 activity was maintained. Tryosinase-catalyzed tyrosine to amine linkage of the IL-12 to chitosan was also shown in the Examples to result in a bioconjugate with IL-12 activity maintained. Other more directed methods of linking IL-12 or another cytokine or growth factor to chitosan covalently may also be used and may have more limited effects on the bioactivity of the conjugated cytokine or growth factor. For example thioester linkage, a peptide linkage via a linking peptide, linkage via engineered lysines or cysteines on the cytokine positioned to limit effects on bioactivity of the cytokine or linkage between amines on the chitosan and the cytokine or growth factor may be used to covalently link chitosan to IL-12 or another cytokine or growth factor. Several of these alternatives are described in the Examples. The linkage via a lysine or cysteine on the cytokine or growth factor may entail making a lysine or cysteine substitution mutation in the cytokine or growth factor. Suitably these substitution mutations are on surface exposed amino acids to allow minimal steric hindrance for the linkage to chitosan and the mutations suitably have minimal effects on the biological activity of the cytokine or growth factor. For example, substitution mutations to position a lysine at one of positions 17, 18, 34, 35, 43, 44 or 248 of IL-12 may be suitable. The chemistry used to link the cytokine or growth factor to chitosan includes, but is not limited to click chemistry, periodate chemistry, maleimide thioether chemistry or thiol chemistry. See Bioconjugate Chem., 2011, 22 (4), pp 551-555. Those of skill in the art will appreciate that other methods may be used to covalently link a cytokine or growth factor to chitosan while maintaining the biological activity of the cytokine or growth factor. Those of skill in the art are also capable of determining if the biological activity of the cytokine-chitosan bioconjugate is sufficient for the intended use of the conjugate.

The chitosan may also be modified prior to linkage to the cytokine or growth factor. The chitosan may be methylated or thiolated to make the linkage chemistry more straightforward. The cytokine or growth factor and the chitosan may be linked directly or through a linker. The linker may be a chemical linker or a peptide linker. The chitosan may be conjugated to the N-terminal alpha amino group, C-terminal alpha carobxyl group, aspartic acid, glutamic acid or cysteine, lysine or tyrosine residues in the cytokine or growth factor.

IL-12 was conjugated to chitosan in the Examples, but a variety of cytokines or growth factors could be conjugated to chitosan to achieve a similar result. For example, cytokines or growth factors including all of the interleukins may be used in the bio-conjugates described herein. The cytokines and growth factors disclosed herein include, but are not limited to, IL-2, IL-12, GM-CSF, IL-1, TNF-α, IFN-γ, IFN-α, IL-10, TGF-β, IL-15, IL-23, IL-27, IL-35 and IL-7. These cytokines/growth factors have been suggested as potential immunotherapeutics but adoption of these cytokines into clinical practice has been limited by toxicity due to systemic administration or other off-target effects of these pleiotropic effectors. Each of these cytokines could be conjugated to chitosan using the methods described above and administered as a pharmaceutical composition similar to IL-12 chitosan in the Examples.

Each of the cytokines may be genetically engineered to add peptide linkers or modify amino acids without negatively impacting the function of the cytokine or growth factor to avoid having covalent linkages to chitosan that diminish or completely alter the activity of the cytokine or growth factor. For example thioester chemistry could be used to link the chitosan to lysine residues on the cytokine. Structural analysis of IL-12 suggested to us that lysine substitutions at amino acid 34 or 248 (R34K or S248K) or a combination thereof would be effective for linkage without altering the activity of the cytokine. Linking via di-sulfide bonds via cysteine substitution is also contemplated and may be used to link the cytokine or growth factor to chitosan.

The chitosan may have a molecular weight between 10kDa and 500kDa, between 100kDa and 400kDa or between 200kDa and 300kDa. Higher molecular weights are likely to lead to less diffusion after administration. In addition the chitosan can be modified and thus have different effects on the stability and diffusion rate of the cytokine-chitosan bioconjugates. The decree of deacetvlation of the chitosan may also be modified in the compositions. Compositions for use in methods of treating a disorder in a subject by administering an effective amount of the compositions are also provided, as set out in the claims. Cells, such as immune cells, may also be contacted with the cytokine/growth factor-chitosan bioconjugate compositions provided herein. In one embodiment, contacting immune cells with the cytokine-chitosan bioconjugate compositions results in an immune response against a cancer or tumor cells and leads to reduced growth or reduced proliferation of the cancer or tumor cells. Suitably the cell is an immune cell, but also may be cells within a turner Suitably the compositions are administered locally to a tumor or to a site near cancer cells and the administration of the compositions effect the local immune response to the cancer or tumor. The cancer may be any cancer, including but not limited to, bladder, breast, colorectal, pancreatic, prostate, renal, lung, melanoma, lymphoma, brain, head and neck, or ovarian cancer. In an alternative embodiment the disorder is a disorder treatable by induction or inhibition of an immune response in a localized area by administration of the compositions described herein. The area could be the site of an infection or immune response such as an allergic or autoimmune response. In an alternative embodiment, the cytokine/growth factor-chitosan bioconjugate is administered with a vaccine or other antigen to act as an adjuvant and stimulate an immune response against one or more antigens. Suitably, the disorder can be treated by induction or repression of an immune response by administration of the compositions described herein. The disorder may further be selected from inflammation, arthritis, Multiple sclerosis, Crohn's disease or others. The subject may be a human or non-human animal such as a domestic animal or agricultural animal. Compositions for use in methods of stimulating an immune response in a subject are also provided, as set out in the claims. The methods include administering the compositions comprising a chitosan-cytokine/growth factor bioconjugate to the subject in an amount effective to stimulate an immune response to the antigen. The antigen may be a protein or peptide antigen or may be part of a vaccine. The subject may be a human or non-human animal such as a domestic animal or agricultural animal and may be administered via any means available to those skilled in the art.

The increased immune response may include an enhanced B cell or T cell mediated immune response and may include increased antibody production, increased class switching or increased cell-mediated killing of infected or cancerous cells.

Cells may be contacted with the composition directly or indirectly *in vivo*, *in vitro*, or *ex vivo.* Contacting encompasses administration to a cell, tissue, mammal, patient, or human. Further, contacting a cell includes adding an agent to a cell culture. Other suitable methods may include introducing or administering the composition to a cell, tissue, mammal, or patient using appropriate procedures and routes of administration as defined below. Tissues include tumors or tissues including cancer cells. Subjects may be human or animal subjects and include cancer patients.

Treating cancer includes, but is not limited to, reducing the number of cancer cells or the size of a tumor in the subject, reducing progression of a cancer to a more aggressive form, maintaining the cancer or tumor in a less aggressive form for a longer period of time, reducing proliferation of cancer cells or reducing the speed of tumor growth, killing of cancer cells, reducing metastasis of cancer cells or reducing the likelihood of recurrence of a cancer in a subject. Treating a subject as used herein refers to any type of treatment that imparts a benefit to a subject afflicted with a disease or at risk of developing the disease, including improvement in the condition of the subject (e.g., in one or more symptoms), delay in the progression of the disease, delay the onset of symptoms or slow the progression of symptoms, etc.

The compounds may be used to make pharmaceutical compositions. Pharmaceutical compositions comprising the compositions described herein and a pharmaceutically acceptable carrier are provided. A pharmaceutically acceptable carrier is any carrier suitable for *in vivo* administration. Examples of pharmaceutically acceptable carriers suitable for use in the composition include, but are not limited to, water, buffered solutions, glucose solutions, oilbased or bacterial culture fluids. Additional components of the compositions may suitably include, for example, excipients such as stabilizers, preservatives, diluents, emulsifiers and lubricants. Examples of pharmaceutically acceptable carriers or diluents include stabilizers such as carbohydrates (e.g., sorbitol, mannitol, starch, sucrose, glucose, dextran), proteins such as albumin or casein, protein-containing agents such as bovine serum or skimmed milk and buffers (e.g., phosphate buffer). Especially when such stabilizers are added to the compositions, the composition is suitable for freeze-drying or spray-drying. The composition may also be emulsified.

The compositions described herein may also be co-administered with another agent such as an anti-cancer therapeutic. The two compositions may be administered in any order, at the same time or as part of a unitary composition. The two may be administered such that one composition is administered before the other with a difference in administration time of 1 hour, 2 hours, 4 hours, 8 hours, 12 hours, 16 hours, 20 hours, 1 day, 2 days, 4 days, 7 days, 2 weeks, 4 weeks or more.

An effective amount or a therapeutically effective amount as used herein means the amount of a composition that, when administered to a subject for treating a state, disorder or condition is sufficient to effect a treatment (as defined above). The therapeutically effective amount will vary depending on the compound, formulation or composition, the disease and its severity and the age, weight, physical condition and responsiveness of the subject to be treated.

The compositions described herein may be administered by any means known to those skilled in the art, including, but not limited to, intratumoral, intravesical, oral, topical, intranasal, intraperitoneal, parenteral, intravenous, intramuscular, subcutaneous, intrathecal, transcutaneous, nasopharyngeal, or transmucosal absorption. Thus the compositions may be formulated as an ingestable, injectable, topical or suppository formulation. The compositions may also be delivered within a liposomal or time-release vehicle or vesicle. Administration of the compositions to a subject in accordance with the invention appears to exhibit beneficial effects in a dose-dependent manner. Thus, within broad limits, administration of larger quantities of the composition is expected to achieve increased beneficial biological effects than administration of a smaller amount. Moreover, efficacy is also contemplated at dosages below the level at which toxicity is seen.

It will be appreciated that the specific dosage administered in any given case will be adjusted in accordance with the compositions being administered, the disease to be treated or inhibited, the condition of the subject, and other relevant medical factors that may modify the activity of the composition or the response of the subject, as is well known by those skilled in the art. For example, the specific dose for a particular subject depends on age, body weight, general state of health, diet, the timing and mode of administration, the rate of excretion, medicaments used in combination and the severity of the particular disorder to which the therapy is applied. Dosages for a given patient can be determined using conventional considerations, e.g., by customary comparison of the differential activities of the compositions of the invention and of a known agent such as an unconjugated cytokine, such as by means of an appropriate conventional pharmacological or prophylactic protocol.

The maximal dosage for a subject is the highest dosage that does not cause undesirable or intolerable side effects. The number of variables in regard to an individual prophylactic or treatment regimen is large, and a considerable range of doses is expected. The route of administration will also impact the dosage requirements. It is anticipated that dosages of the composition will reduce symptoms of the condition at least 10%, 20%, 30%, 40%, 50%, 60%, 70%, 80%, 90% or 100% compared to pre-treatment symptoms or symptoms is left untreated. It is specifically contemplated that pharmaceutical preparations and compositions may palliate or alleviate symptoms of the disease without providing a cure, or, in some embodiments, may be used to cure the disease or disorder.

Suitable effective dosage amounts for administering the compositions may be determined by those of skill in the art, but typically range from about 1 microgram to about 100,000 micrograms per kilogram of body weight weekly, although they are typically about 1,000 micrograms or less per kilogram of body weight weekly. Smaller doses may be required because the compositions are not diffusing into the surrounding and have a more targeted effect as compared to non-conjugated counterpart compositions, In some embodiments, the effective dosage amount ranges from about 10 to about 10,000 micrograms per kilogram of body weight weekly. In another embodiment, the effective dosage amount ranges from about 50 to about 5,000 micrograms per kilogram of body weight weekly. In another embodiment, the effective dosage amount ranges from about 75 to about 1,000 micrograms per kilogram of body weight weekly. The effective dosage amounts described herein refer to total amounts administered, that is, if more than one composition is administered, the effective dosage amounts correspond to the total amount administered. The composition can be administered as a single dose or as divided doses. For example, the composition may be administered two or more times separated by 4 hours, 6 hours, 8 hours, 12 hours, a day, two days, three days, four days, one week, two weeks, or by three or more weeks.

The present disclosure is not limited to the specific details of construction, arrangement of components, or method steps set forth herein. The compositions and methods disclosed herein are capable of being made, practiced, used, carried out and/or formed in various ways that will be apparent to one of skill in the art in light of the disclosure that follows. The phraseology and terminology used herein is for the purpose of description only and should not be regarded as limiting to the scope of the claims. Ordinal indicators, such as first, second, and third, as used in the description and the claims to refer to various structures or method steps, are not meant to be construed to indicate any specific structures or steps, or any particular order or configuration to such structures or steps. All methods described herein can be performed in any suitable order unless otherwise indicated herein or otherwise clearly contradicted by context The use of any and all examples, or exemplary language (e.g., "such as") provided herein, is intended merely to facilitate the disclosure and does not imply any limitation on the scope of the disclosure unless otherwise claimed. No language in the specification, and no structures shown in the drawings, should be construed as indicating that any non-claimed element is essential to the practice of the disclosed subject matter. The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements,

Recitation of ranges of values herein are merely intended to serve as a shorthand method of referring individually to each separate value falling within the range, unless otherwise indicated herein, and each separate value is incorporated into the specification as if it were individually recited herein. For example, if a concentration range is stated as 1 % to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this disclosure. Use of the word "about" to describe a particular recited amount or range of amounts is meant to indicate that values very near to the recited amount are included in that amount, such as values that could or naturally would be accounted for due to manufacturing tolerances, instrument and human error in forming measurements, and the like. All percentages referring to amounts are by weight unless indicated otherwise.

No admission is made that any reference, including any non-patent or patent document cited in this specification, constitutes prior art. In particular, it will be understood that, unless otherwise stated, reference to any document herein does not constitute an admission that any of these documents forms part of the common general knowledge in the art in the United States or in any other country. Any discussion of the references states what their authors assert, and the applicant reserves the right to challenge the accuracy and pertinence of any of the documents cited herein. The present disclosure shall control in the event there are any disparities between any definitions and/or description found in the cited references.

The following examples are meant only to be illustrative and are not meant as limitations on the scope of the invention or of the appended claims.

### EXAMPLES

Our group has pioneered the use of chitosan-based solutions for the local delivery of recombinant cytokines and antigens [6, 38, 39, 88-91], Chitosan is an abundant, natural polysaccharide derived primarily from the exoskeletons of crustaceans [92]. It is an unbranched copolymer of glucosamine and N-acetylglucosamine units linked by β(1-4) glycosidic bonds (**Figure 1**). The use of water soluble polysaccharides, like chitosan, avoids the use of cytokine-denaturating organic solvents. In vivo, chitosan is safely degraded into excretable glucosamine and N-acetylglucosamine fragments by lysozyme, glucosaminidase, lipase and other endogenous human enzymes. The rate of degradation can be controlled via chitosan concentration, molecular weight (MW), injection volume and N-acetylglucosamine: glucosamine ratio.

***Chitosan Solution Enhances Local Retention and Activity of Delivered Cytokines:*** Our published studies demonstrated that simple, viscous chitosan solutions are able to significantly increase the local retention of co-formulated GM-CSF [88] and IL-12 [6]. In particular, i.t. injections of IL-12 alone dissipated quickly and became undetectable within 24 to 48 hrs (**Figure 2**). In contrast, by formulating IL-12 in chitosan solution, IL-12 was detectable for up to 6 days [6]. We believe that a combination of viscous and electrostatic interactions hinder the ability of cytokines to diffuse out of the chitosan solution and thus form a slow-release delivery system. The nature of non-covalent chitosan/cytokine interactions and the mechanism of cytokine release is the subject of ongoing studies in our lab.

Our data also showed that chitosan/cytokine depots also increased immunological activity versus saline-based cytokine injections. Specifically, a single chitosan/GM-CSF injection outperformed four daily injections of GM-CSF alone in terms of increasing the number and functionality of dendritic cells in draining lymph nodes. In vaccination experiments, chitosan/GM-CSF was superior to either chitosan or CM-CSF alone in enhancing antigen-specific CD4⁺ proliferation, peptide-specific CD8⁺ pentamer staining and cytotoxic T cell lysis [88]. Similarly, chitosan/IL-12 formulations outperformed IL-12 alone in eradicating 80-100% of aggressive, established solid tumors (MC38 and Panc02) [6] and 88-100% orthotopic, superficial bladder tumors (MB49) [38]. Interestingly, intravesical chitosan/IL-12 immunotherapy was found to induce systemic tumor-specific immunity which conferred complete protection from a distant s.c. tumor rechallenge. More recent unpublished data demonstrate that i.t injections of chitosan/lL-12 prior to tumor resection are capable of eliminating metastasis and extending survival (**Figure 3**) in an aggressive, highly metastatic model of breast cancer.

***Limitations of chitosan*/*****cytokine mixtures:*** Although we have successfully demonstrated that simple chitosan/cytokine mixtures are effective at increasing local cytokine retention and activity, we determined that most of the delivered cytokine leaches out of the chitosan depot and into the circulation. In fact, serum IL-12 and IFN-γ levels following i.t. injection of either IL-12 alone or chitosan/IL-12 mixtures were similar [38]. Because these data may limit the clinical application of our simple mixture platform due to concerns over IL-12-mediated toxicity, we developed a new delivery technology capable of preventing IL-12 dissemination.

***Novel IL-12-chitosan bioconjugates:*** The amine functional group of chitosan's glucosamine residues (**Figure 1**) allows for facile chemical attachment of a variety of side chain moieties including proteins. As a result, we hypothesized that cytokines could be covalently conjugated to chitosan. Direct conjugation has two advantages over the previous simple chitosan/IL-12 mixtures. First, because the diffusion of macromolecules is inversely related to molecular size, by conjugating IL-12 (MW=75kDa) to a comparatively large chitosan molecule (MW=100-500kDa), the effective molecular weight of the cytokine can be increased up to 6-7 fold. As a result, diffusive transport and therefore systemic dissemination of cytokines would be drastically reduced if not eliminated altogether. Second, after i.t. injection, highly polycationic chitosan molecules interact electrostatically with negatively charged extracellular matrices and cell membranes [93]. Consequently, cytokines are effectively "anchored" to the injection site by chitosan's polycationic charge.

Our group has found that locally administered mixtures of chitosan and IL-12 can induce the complete regression of pancreatic adenocarcinomas [6], colon adenocarcinomas [6], bladder carcinomas [38], metastatic mammary carcinomas, renal cell carcinomas, melanomas and prostate adenocarcinomas. While this simple mixture platform is effective in retaining significant amounts of IL-12 in the tumor microenvironment, it does not prevent systemic dissemination of the majority of IL-12. It is estimated that as much as 75% of the injected IL-12 was absorbed into circulation and resulted in a concomitant spike in serum IFN-γ [38], While a weekly dosing schedule of chitosan/IL-12 mixtures is not expected to induce clinical toxicity [97], novel technologies which minimize or eliminate systemic IL-12 exposure are more translatable.

Thus, the proposed project will develop and evaluate a novel delivery platform based on the conjugation of IL-12 to chitosan. This strategy hinders IL-12 dissemination by: 1) increasing the effective size of IL-12; and 2) anchoring IL-12-chitosan bioconjugates to a local injection site through bioadhesive interactions. This approach has the potential to maintain high concentrations of IL-12 in the tumor following i.t. administration with minimal leakage into the circulation.

***Overexpression and purification of recombinant human IL-12:*** The plans of our initial application to manufacture IL-12 using bacterial (*E*. *coli*) and yeast (*Pichia Pastoris*) expression systems were not successful due to the extensive glycosylation required for IL-12 expression and bioactivity. Fortunately, our collaborator, Dr. Barbara Felber, Senior Investigator, Vaccine Branch, NCI, has developed human embryonic kidney (HEK293) cells expressing murine IL-12 (mIL-12) and human IL-12 (buIL-12).

To avoid the potential generation of antigenic epitopes, none of the provided recombinant IL-12 constructs contains extrinsic affinity tags for purification. Careful examination of the amino acid sequence of IL-12 revealed that the p40 subumt of both mIL-12 and huIL-12 contain amino segments which can potentially bind to glycosaminoglycans such as, heparin. Interestingly, isothermal titration calorimetry (ITC) data showed that human IL-12 has strong binding affinity to heparin (K_{d} ∼ 70 µM, **Figure 4A**). Based on the high binding affinity of IL-12 to heparin, an affinity chromatography (using heparin-sepharose) based purification protocol was designed to purify human IL-12. IL-12 was observed to elute as discrete peak at 500 mM NaCl (**Figure 4B****)**. SDS-PAGE gel analysis revealed that IL-12 was pure (>95%, **Figure 4C, D**). The yield of the purified protein is ∼6.5 mg/20 mL of culture supernatant.

The acquisition of IL-12-expressing HEK293 clones and the identification of heparin-binding motifs on IL-12 are helpful advances needed to move to mammalian expression systems. In addition, the newly identified heparin-binding motifs allow for facile purification of authentic IL-12, as well as planned IL-12 mutants, from culture supernatants without the involvement of affinity tags or laborious multi-step procedures. See Jayanthi et al. Protein Expr Purif (2014) 102: 76-84.

***Validation* of** ***IL-12-chmosan bioconjugates:*** Additional preliminary data demonstrate the successful non-specific conjugation of mIL-12 to chitosan via carbodiimide cross-linking. Briefly, 1-ethyl-3-(3-dimethylaminopropyl) carbodiimide (EDAC) was used to activate carboxyl groups on IL-12 which in turn reacted with N-hydroxysuccinamide (NHS) stabilized amine groups on chitosan to form a peptide bond. The conjugation reaction was carried out at pH 5.0 for 6 hours after which IL-12-chitosan bioconjugates were isolated via dialysis. In vitro bioactivity was verified by quantifying proliferation of IL,12-sensitive 2D6 cells (**Figure 5A**). As expected due to non-specific conjugation, a significant amount, approximately 35%, of IL-12 bioactivity was lost. Possible causes include loss of unreacted IL-12, direct inactivation of IL-12 by cross-linking reagents, or indirect inactivation by conjugation of IL-12 to chitosan in an inaccessible orientation. Nevertheless, it is reasonable to assume that any loss in IL-12 bioactivity can be counteracted by simply increasing doses of IL-12-chitosan used in subsequent immunotherapy studies. To control for the possibility that IL-12-chitosan-induced proliferation was due to unconjugated IL-12, a mixture of IL-12 and chitosan was subjected to dialysis but not the conjugation reaction. Unconjugated IL-12 and chitosan did not induce 2D6 proliferation indicating that the dialysis purification successfully removed free, unreacted IL-12.

In vivo bioactivity of IL-12-chitosan bioconjugates was verified by documenting tumor regression following i.t. administration (**Figure 5B**). I.t IL-12-chitosan immunotherapy mediated tumor regression in 4 of 4 treated mice, including 2 complete regressions. These data demonstrate that first generation, non-specifically conjugated IL-12-chitosan constructs are not only active, but also promising antitumor agents. The proposed methods of specific conjugation of IL-12 to chitosan will attempt to improve upon the bioactivity of IL-12-chitosan bioconjugates through more controlled and site-specific conjugation protocols.

**IL-12 maintains bioactivity when linked to chitosan in a variety of ways.** Chitosan (degree of deacetylation >90%) was purchased from Primex (Siglufjordur, Iceland) and purified before use. Hydrochloric acid, sodium hydroxide, I -ethyl-3-(3-dimethylaminopropyl) carbodiimide (EDAC), N - hydroxysuccinimide (NHS), and mushroom tyrosinase were purchased from Sigma (St. Louis, MO). Sulfosuccinimidyl 4-(N-maleimidomethyl)cyclohexane-1-carboxylate (Sulfo-SMCC) was purchased from Life Technologies (Green Island, NY).

### Carbodiimide mediated carboxyl-to-amine reactive group crosslinking:

Primary amine groups on chitosan were crosslinked with free carboxylic acid groups on proteins through carbodiimide mediated peptide bonding. Briefly, 1mg of chitosan was dissolved in 0.1M HCL and pH adjusted to 5.4 to 5.6 using 1M NaOH. 1mg of EDAC and NHS were added to 250µl of chitosan stock solution and mixed before adding 50µl of 1mg/ml IL-12 solution. The mixture was lest at room temperature for 6 hours for optimal conjugation efficiency. After 6 hours the chitosan-IL-12 conjugates were dialyzed using 100KD dialysis membrane to remove unreacted chemicals and freeze dried before further use.

### Maleimide thioether mediated amine-to-sulfhydryl crosslinking:

Reactive amine groups on IL-12 were crosslinked with sulfhydryl groups on thiolated chitosan through maleimide NHS ester mediated amine-sulfhydryl crosslinking. Briefly, amine-to-sulfhydryl crosslinker Sulfo-SMCC was added to 50µg of XL-12 in 50µl deionized water at 80X molar access. After 30min incubation at room temperature the reaction mixture was desalted and added to 250µg of thiolated chitosan. After 30min, the chitosan-IL-12 conjugates were dialyzed using 100KD dialysis membrane to remove unreacted compounds and freeze dried before further use.

### Tyrosinase-catalyzed tyrosine-to-amine crosslinking:

Reactive O-quinone created by tyrosinase catalyzed oxidation of tyrosine on IL-12 was used for crosslinking to chitosan via the primary amine group. Briefly, chitosan was dissolved in 20mM HCL and pH adjusted to 6.0 using 1M NaOH to get 0.1% (w/v) chitosan solution. Tyrosinase was diluted in PBs (pH 6.5) to achieve specific activity of 120U/ml. Equal volumes of tyrosinase and chitosan stock solutions were mixed and left at room temperature. 50µg of IL-12 was added to the reaction mixture and mixed at room temperature. After 8 hours of incubaton, the chitosan-IL-12 conjugates were dialyzed using 100KD dialysis membrane to remove unreacted compounds and freeze dried before further use.

### Bioactivity of IL-12 chitosan conjugates:

Influence of non-specific conjugation or IL-12 onto chitosan on IL-12 bioactivity was determined by quantifying the proliferation of IL-12 responsive 2D6 cell line. In brief, cultured 2D6 T-cells were seeded in a 96 well plate at 20,000 cells/well. IL-12 was added to achieve final concentrations of 0.2 ng/mL, 0.04 ng/ml, and 0,008 ng/ml. Un-conjugated IL-12 and culture media alone served as controls. After a 24 hour incubation, IL-12-dependent proliferation of 2D6 cells was quantified via MTT based proliferation assay. The results are shown in Figure 6 and demonstrate that the various means of linking the IL-12 to the chitosan result in various activity levels. Carboxyl to amine and tyrosine to amine linkage of the IL-12 to chitosan resulted in better IL-12 activity.

### Bibliography

[3] Lotze MT, Zitvogel L, Campbell R, Robbins PD, Elder E, Haluszczak C, et al. Cytokine gene therapy of cancer using interleukin-12: murine and clinical trials. Ann N Y Acad Sci 1996;795:440-454.
[4] Nastala CL, Edington HD, McKinney TG, Tahara H, Nalesnik MA, Brunda MJ, et al. Recombinant IL-12 administration induces tumor regression in association with IFN-gamma production. J Immunol 1994;153:1697-1706.
[5] Zitvogel L, Tahara H, Robbins PD, Storkus WJ, Clarke MR, Nalesnik MA, et al. Cancer immunotherapy of established tumors with IL-12. Effective delivery by genetically engineered fibroblasts. J Immunol 1995;155:1393-1403.
[6] Zaharoff DA, Hance KW, Rogers CJ, Schlom J, Greiner JW. lntratumoral immunotherapy of established solid tumors with chitosan/IL-12. J Immunother 2010;33:697-705.
[38] Zaharoff DA, Hoffman BS, Hooper HB, Benjamin CJ, Jr., Khurana KK, Hance KW, et al. Intravesical immunotherapy of superficial bladder cancer with chitosan/interleukin-12. Cancer Res 2009;69:6192-6199.
[39] Yang L, Zaharoff DA. Role of chitosan co-formulation in enhancing interleukin-12 delivery and antitumor activity. Biomaterials 2013;34:3828-3836.
[88] Zaharoff DA, Rogers CJ, Hance KW, Schlom J, Greiner JW. Chitosan solution enhances the immunoadjuvant properties of GM-CSF. Vaccine 2007;25:8673-8686.
[89] Zaharoff DA, Rogers CJ, Hance KW, Schlom J, Greiner JW. Chitosan solution enhances both humoral and cell-mediated immune responses to subcutaneous vaccination. Vaccine 2007;25:2085-2094.
[90] Heffeman MJ, Zaharoff DA, Fallon JK, Schlom J, Greiner JW. In vivo efficacy of a chitosan/IL-12 adjuvant system for protein-based vaccines. Biomaterials 2011;32:926-932.
[91] Koppolu B, Zaharoff DA. The effect of antigen encapsulation in chitosan particles on uptake, activation and presentation by antigen presenting cells. Biomaterials 2013;34:2359-2369.
[92] Illum L. Chitosan and its use as a pharmaceutical excipient. Pharm Res 1998;15:1326-1331.
[93] Morris G, Kok S, Harding S, Adams G. Polysaccharide drug delivery systems based on pectin and chitosan. Biotechnol Genet Eng Rev 2010;27:257-284.
[94] Singla AK, Chawla M. Chitosan: some pharmaceutical and biological aspects-an update. J Pharm Pharmacol 2001:53:1047-1087.
[95] Pittler MH, Ernst E. Dietary supplements for body-weight reduction; a systematic review. Am J Clin Nutr 2004;79:529-536.
[96] Mhurchu CN, Dunshea-Mooij C, Bennett D, Rodgers A. Effect of chitosan on weight loss in overweight and obese individuals: a systematic review of randomized controlled trials. Obes Rev 2006;6:35-42.
[97] van Herpen CM, van der Laak JA, de Vries IJ, van Krieken JH, de Wilde PC, Balvers MG, et al. Intratumoral recombinant human interleukin-12 administration in head and neck squamous cell carcinoma patients modifies locoregional lymph node architecture and induces natural killer cell infiltration in the primary tumor. Clin Cancer Res 2005;11:1899-1909.
[98] Yoon C, Johnston SC, Tang J, Stahl M, Tobin JF, Somers WS. Charged residues dominate a unique interlocking topography in the heterodimeric cytokine Interleukin-12. EMBO J 2000;19:3530-3541.

## Claims

1. A composition comprising chitosan covalently linked to IL-12, wherein the covalent linkage is a tyrosine to amine linkage of the IL-12 to chitosan, is between amine groups on the chitosan and carboxyl groups on the IL-12, or is between sulfhydryl groups in cysteine-substituted IL-12 and on the chitosan which is thiolated, and wherein the IL-12 is biologically active.

2. The composition of claim 1, wherein the IL-12 is covalently linked to the chitosan using a chemistry selected from the group consisting of click chemistry, periodate chemistry, maleimide thioether chemistry and thiol chemistry to generate the covalent linkage.

3. The composition of any one of claims 1-2, wherein the chitosan has a molecular weight between 10 kDa and 500 kDa, optionally between 100 kDa and 400 kDa and optionally between 200 kDa and 300 kDa.

4. The composition of any one of the preceding claims, wherein the chitosan is thiolated or methylated.

5. A pharmaceutical composition comprising the composition of any one of claims 1-4 and a pharmaceutically acceptable carrier.

6. The composition of any one of claims 1-5 for use in a method of treating a disorder in a subject, the method comprising administering the composition to the subject in an amount effective to treat the disorder.

7. The composition for use of claim 6, wherein the subject is human.

8. The composition for use of claim 6 or claim 7, wherein the disorder is localized to an area and the composition is administered locally.

9. The composition for use of any one of claims 6-8, wherein the disorder is cancer.

10. The composition of any one of claims 1-5 for use in a method of stimulating an immune response in a subject, the method comprising administering the composition and an antigen to the subject in an amount effective to stimulate an immune response to the antigen.,

11. The composition for use of claim 10, wherein the antigen is part of a vaccine.

12. The composition for use of claim 10 or claim 11, wherein the subject is human.

13. The composition for use of any one of claims 6-12, wherein the composition is administered via a method selected from the group consisting of intratumoral, intravesicular, oral, topical, intranasal, intraperitoneal, parenteral, intravenous, intramuscular, subcutaneous, intrathecal, and transcutaneous administration.

## Patentansprüche

1. Zusammensetzung, die kovalent an IL-12 gebundenes Chitosan umfasst, wobei die kovalente Bindung eine Tyrosin-Amin-Bindung des IL-12 an das Chitosan, zwischen Aminogruppen am Chitosan und Carboxylgruppen am IL-12 oder zwischen Sulfhydrylgruppen in Cystein-substituiertem IL-12 und im Chitosan, das thioliert ist, ist und wobei das IL-12 biologisch aktiv ist.

2. Zusammensetzung nach Anspruch 1, wobei das IL-12 mittels einer aus der aus Click-Chemie, Periodat-Chemie, Maleinimid-Thioether-Chemie und Thiolchemie ausgewählten Chemie kovalent an das Chitosan gebunden ist, um die kovalente Bindung herzustellen.

3. Zusammensetzung nach einem der Ansprüche 1 bis 2, wobei das Chitosan ein Molekulargewicht zwischen 10 kDa und 500 kDa, gegebenenfalls zwischen 100 kDa und 400 kDa und gegebenenfalls zwischen 200 kDa und 300 kDa, aufweist.

4. Zusammensetzung nach einem der vorangegangenen Ansprüche, wobei das Chitosan thioliert oder methyliert ist.

5. Pharmazeutische Zusammensetzung, die eine Zusammensetzung nach einem der Ansprüche 1 bis 4 und einen pharmazeutisch annehmbaren Träger umfasst.

6. Zusammensetzung nach einem der Ansprüche 1 bis 5 zur Verwendung in einem Verfahren zur Behandlung einer Störung bei einem Individuum, wobei das Verfahren das Verabreichen der Zusammensetzung an das Individuum in einer zur Behandlung der Störung wirksamen Menge umfasst.

7. Zusammensetzung zur Verwendung nach Anspruch 6, wobei das Individuum ein Mensch ist.

8. Zusammensetzung zur Verwendung nach Anspruch 6 oder 7, wobei die Störung in einem Bereich lokalisiert ist und die Zusammensetzung lokal verabreicht wird.

9. Zusammensetzung zur Verwendung nach einem der Ansprüche 6 bis 8, wobei die Störung Krebs ist.

10. Zusammensetzung nach einem der Ansprüche 1 bis 5 zur Verwendung in einem Verfahren zur Stimulierung einer Immunantwort in einem Individuum, wobei das Verfahren das Verabreichen der Zusammensetzung und eines Antigens an das Individuum in einer zur Stimulierung einer Immunantwort auf das Antigen wirksamen Menge umfasst.

11. Zusammensetzung zur Verwendung nach Anspruch 10, wobei das Antigen Teil einer Vakzine ist.

12. Zusammensetzung zur Verwendung nach Anspruch 10 oder Anspruch 11, wobei das Individuum ein Mensch ist.

13. Zusammensetzung zur Verwendung nach einem der Ansprüche 6 bis 12, wobei die Zusammensetzung mittels eines Verfahrens verabreicht wird, das aus der aus intratumoraler, intravesikulärer, oraler, topischer, intranasaler, intraperitonealer, parenteraler, intravenöser, intramuskulärer, subkutaner, intrathekaler und transkutaner Verabreichung bestehenden Gruppe ausgewählt ist.

## Revendications

1. Composition comprenant du chitosane lié de manière covalente à de l'IL-12, dans laquelle la liaison covalente est une liaison tyrosine à amine de l'IL-12 au chitosane, est entre des groupes amine sur le chitosane et des groupes carboxyle sur l'IL-12, ou est entre des groupes sulfhydryle dans l'IL-12 à substitution cystéine et sur le chitosane qui est thiolé, et dans laquelle l'IL-12 est biologiquement active.

2. Composition selon la revendication 1, dans laquelle l'IL-12 est liée de manière covalente au chitosane en utilisant une chimie choisie dans le groupe consistant en la chimie clic, la chimie périodate, la chimie thioéther-maléimide et la chimie thiol pour générer la liaison covalente.

3. Composition selon l'une quelconque des revendications 1 à 2, dans laquelle le chitosane a un poids moléculaire compris entre 10 kDa et 500 kDa, facultativement entre 100 kDa et 400 kDa et facultativement entre 200 kDa et 300 kDa.

4. Composition selon l'une quelconque des revendications précédentes, dans laquelle le chitosane est thiolé ou méthylé.

5. Composition pharmaceutique comprenant la composition selon l'une quelconque des revendications 1 à 4 et un support pharmaceutiquement acceptable.

6. Composition selon l'une quelconque des revendications 1 à 5 à utiliser dans un procédé de traitement d'un trouble chez un sujet, le procédé comprenant l'administration de la composition au sujet en une quantité efficace pour traiter le trouble.

7. Composition à utiliser selon l'une quelconque des revendications 1 à 6, dans laquelle le sujet est être humain.

8. Composition à utiliser selon la revendication 6 ou la revendication 7, dans laquelle le trouble est localisé dans une zone et la composition est administrée localement.

9. Composition à utiliser selon l'une quelconque des revendications 6 à 8, dans laquelle le trouble est un cancer.

10. Composition selon l'une quelconque des revendications 1 à 5 à utiliser dans un procédé de stimulation d'une réponse immunitaire chez un sujet, le procédé comprenant l'administration de la composition au sujet en une quantité efficace pour stimuler une réponse immunitaire à l'antigène.

11. Composition à utiliser selon la revendication 10, dans laquelle l'antigène est une partie d'un vaccin.

12. Composition à utiliser selon la revendication 10 ou la revendication 11, dans laquelle le sujet est être humain.

13. Composition à utiliser selon l'une quelconque des revendications 6 à 12, dans laquelle la composition est administrée via un procédé choisi dans le groupe comprenant une administration intratumorale, intravésiculaire, orale, topique, intranasale, intrapéritonéale, parentérale, intraveineuse, intramusculaire, sous-cutanée, intrathécale et transcutanée.
